# EUROPEAN PATENT SPECIFICATION

(11) **EP 2 220 142 B1**
(45) Date of publication and mention of the grant of the patent: **24.02.2016**
(21) Application number: 09812507.3
(22) Date of filing: 06.07.2009
(51) Int. Cl.: C08G 63/90, C08G 63/88, C08G 63/89, C08G 63/06, C08G 63/02

(54) **A METHOD OF EXTRACTING AND PURIFYING POLYHYDROXY ALKANOATE BIOPLASTIC**
VERFAHREN ZUR EXTRAKTION UND REINIGUNG VON POLYHYDROXY-ALKANOAT-BIOKUNSTSTOFFEN
PROCEDE D'EXTRACTION ET DE PURIFICATION DE BIOPLASTIQUE DE POLYHYDROXY-ALCANOATES

(30) Priority: 18.12.2008 MY 0805129
(43) Date of publication of application: 25.08.2010
(73) Proprietor: Universiti Putra Malaysia, 43400 Selangor Darul Ehsan (MY); Voon, Phooi Tee, 43400 Selangor Darul Ehsan (MY); Phang, Lai Yee, 43400 Selangor Darul Ehsan (MY); Shirai, Yoshihito, 43400 Selangor Darul Ehsan (MY)
(72) Inventor: HASSAN, Mohd. Ali, 43400 Selan or Darul Ehsan (MY)
(74) Representative: Hamer, Christopher K.
(86) International application number: PCT/MY2009/000094
(87) International publication number: WO 2010/071399

(56) References cited:
- US-A1- 2003 186 398
- US-A1- 2008 118 963
- US-B2- 7 393 668
- US-B2- 7 393 668
- US-B2- 7 435 567
- US-B2- 7 435 567

## Description

### FIELD OF INVENTION

The present invention relates to a method of extracting and purifying polyhydroxyalkanoate (PHA), a biodegradable plastic produced by bacterial cells.

### BACKGROUND OF INVENTION

PHA is a biodegradable, biocompatible, microbial thermoplastic which has the potential to replace petroleum-derived thermoplastics. The molecular weight of PHA is in the range of 50-1000 kDa with polymer characteristics that are similar to conventional plastics such as polypropylene. Moreover, PHA is produced from various renewable resources such as carbohydrates, organic acids, carbon dioxide and organic wastes.

However, the use of biologically produced polymers is currently limited because of high production costs. P (3HB-co-3HV), a co-polymer that is commercially available currently costs more than US$5 per kilogram and is more expensive than polypropylene which is only US$1-2 per kilogram. Significant contributors to the cost of production are the productivity of PHA by the bacterial strain, carbon source and downstream processing.

The difficulty of PHA recovery from microorganisms has been the primary obstacle to its commercial exploitation. The majority of separation processes that had been carried out involved the extraction of PHA from cells is not only costly but also not environmentally-friendly. Hence, a simpler, economical and environmentally friendly method of recovering intracellular PHA from microorganisms is needed to provide alternative to the conventional methods.

### SUMMARY OF THE INVENTION

This invention describes a method of extracting and purifying the bioplastic polyhydroxyalkanoate (PHA) from PHA-rich bacterial cells. The method includes the steps of (a) providing dried pellets containing PHA-rich bacterial cells, (b) pulverizing the dried pellets, (c) partially dissolving the ground pellets obtained from step (b) with an alkaline solution, (d) homogenizing the PHA-enriched alkaline solution obtained from step (c), (e) separating the non-PHA cellular material (NPCM) from the PHA-enriched alkaline solution and (f) recovering the PHA granules from the PHA-enriched alkaline solution.

The present invention consists of several novel features and a combination of parts hereinafter fully described and illustrated in the accompanying description, it being understood that various changes in the details may be made without departing from the scope of the invention or sacrificing any of the advantages of the present invention.

### DETAILED DESCRIPTION OF PREFERRED EMBODIMENTS

The present invention relates to a method of extracting and purifying PHA. Hereinafter, this specification will describe the present invention according to the preferred embodiments of the present invention. However, it is to be understood that limiting the description to the preferred embodiments of the invention is merely to facilitate discussion of the present invention and it is envisioned that those skilled in the art may devise various modifications and equivalents without departing from the scope of the appended claims.

The embodiments disclosed herein relate to novel methods for the extraction and recovery of PHA polymer from biomass materials, wherein the biomass materials are derived from PHA-producing microorganisms.

There have been a number of reports describing the solvent or halogenated extraction of PHA from biomass, primarily from PHA-producing microorganisms. The most common chemical used are chloroform, methylene chloride or 1,2-dichloroethane. Through this recovery method, more than 90% of the polymer in cells can be obtained. However, due to its chemical property that is hazardous to the environmental and costly operation, we have found that the methods are economically not feasible, particularly under conditions desired for commercial scale processing and extraction of PHA.

The methods of the present invention are applicable to the recovery of PHA polymers produced by microorganisms either naturally or through genetic engineering, or PHAs that are synthetically produced. PHA is a general class of microbial polyester which are biodegradable and biocompatible properties. The physical properties can be regulated by varying the composition of the copolymers. PHA appears as discrete granules in the bacterial cells. PHAs are intracellular carbon and energy reserve materials accumulated by a wide range of bacteria. The PHA compounds are polyesters of hydroxyalkanoic acids. The monomers in the polymer are all In the D(-) configuration, implying the specificity in the biosynthetic route. PHA has molecular weights in the range of 200 to 3000 kDa, depending on the source of material.

Owing to its location which is intracellular, the first step in extraction of PHA is cell disruption. Cell disruption comprised of mechanical and non-mechanical methods. Complete destruction of the wall and release of all intracellular components require destruction of the strength-providing components of the wall, i.e. peptidoglycan in Gram-negative bacteria. The cell disruption techniques with the best industrial potential are the mechanical methods. The characteristic monitored during the mechanical disruption include the efficiency of disruption (measured by amount of protein released, activity of enzymes or number of surviving cells) as well as physical properties, very important for downstream processing. Different characteristics of the disintegrated cells caused by different mechanical devices (e.g. size of cell debris, particle size distribution, viscosity) influence the efficiency of separation techniques used for the isolation of a desired product.

It has been identified in the present invention by combining the chemical and mechanical processes can improve the recovery of PHA. Chemical treatment such as alkaline solution increases the permeability of the cells, causing partial protein released. While this may suffice in the partial separation of NPCM, more complete rupture may be required for the release of granular products such as bacterial inclusion bodies and PHA. Mechanical disruption poses high energy requirements and frequently causes the micronization (particle size reduction) of cell debris, thereby hindering further separations. On the combination of a chemical pretreatment such as alkaline solution, increased monovalent cation concentration with subsequent high pressure homogenization, it has been shown that the disruption is greatly enhanced. Maximum protein released is approached on a single pass, at reduced operating pressure.

In a further embodiment of the present invention, different concentrations of NaOH were tested on the effectiveness of intracellular PHA recovery at 30°C for 1 hour. For 0.1 molar NaOH treatment, protein released was 0.26 g/g biomass. Protein released increased to 0.42 g/g biomass at 0.5 molar NaOH. Besides, PHA purity obtained achieved 46% after treatment with 0.1 molar NaOH. Further increment in NaOH molarity up to 1 molar increased the protein released and PHA purity up to 67% and 0.54 g/g biomass, respectively. Thus, protein released and purity of PHA increased with the increase of NaOH concentration. On the other hand, the yield of PHA decreased with increased NaOH concentration from 0.26 g/g biomass or 80% yield at 0.1 molar NaOH to 0.20 g/g biomass or 64% yield at 1 molar NaOH.

### EXAMPLE

A culture medium containing PHA-rich cells and growth broth was separated using centrifugation method at 3500 rpm for 10 minutes. Pellet produced was rinsed with distilled water twice to remove residual fatty acids such as acetic and propionic acids. The supernatant was then discarded. The wet-pellet was dried in oven for 24 hours at 60°C. The dried biomass then was grounded by using mortar and pestle to make fine cells for further processing or stored at 4°C.

5 g/L of biomass containing PHA was treated with 0.2 molar of NaOH for 60 minutes at 30°C. Homogenization was then carried out immediately. Homogenization was done by using rotor-stator type homogenizer at a fixed speed of 8000 rpm, the lowest speed to conserve energy and cost. The temperature of the sample was restricted to not exceed 35°C, thus cooling of sample with ice or cold water was required to prolong homogenization time. The final PHA obtained was rinsed twice with deionized water, centrifuged at 3500 rpm for 10 minutes and air-dried.

## Claims

1. A method of extracting and purifying polyhydroxyalkanoate (PHA) from PHA-rich bacterial cells, the method incudes the steps of:
(a) providing dried pellets containing PHA-rich bacterial cells;
(b) pulverizing the dried pellets;
(c) dissolving the ground pellets obtained from step (b) with an alkaline solution;
(d) homogenizing the PHA-enriched alkaline solution obtained from step (c);
(e) separating the non-PHA cellular material (NPCM) from the PHA-enriched alkaline solution; and
(f) recovering PHA granules from the PHA-enriched alkaline solution.

2. The method of claim 1, wherein the PHA is selected from the group consisting of poly(hydroxybutyrate-co-hydroxyvalerate) (PHBV), and polymers and copolymers of hydroxyterminated polyhydroxybutyrate (PHB-OH).

3. The method of claim 1, wherein the bacterial cells are PHA-producing microorganisms.

4. The method of claim 1, wherein the dried pellets are produced from PHA-rich bacterial cells after separation using centrifugation.

5. The method of claim 4, wherein the dried pellets are produced at approximately 60°C for about 24 hours.

6. The method of claim 1, wherein the alkaline solution Is 0.2 molar sodium hydroxide (NaOH).

7. The method of claim 6, wherein the alkaline treatment of PHA-enriched solution using the alkaline solution is conducted for about 60 minutes.

8. The method of claim 1, wherein step (d) is conducted at a speed of 8000 rpm.

9. The method of claim 8, wherein the homogenization of the PHA-enriched alkaline solution is conducted for about 18 minutes.

10. The method of claim 1, wherein NPCM is separated from PHA-enriched alkaline solution by centrifugation.

11. The method of claim 10, wherein centrifugation the NPCM Is separated twice at approximately 3500 rpm for about 10 minutes.

12. The method of claim 1, wherein the PHA granules are recovered from alkaline solution by twice rinsing with deionized water in between the centrifugation process.

13. The method of claim 1, wherein the PHA granules are produced by drying.

## Patentansprüche

1. Verfahren zum Extrahieren und Reinigen von Polyhydroxyalkanoat (PHA) aus PHA-reichen Bakterienzellen, wobei das Verfahren die folgenden Schritte einschließt:
(a) Bereitstellen getrockneter Pellets, enthaltend PHAreiche Bakterienzellen;
(b) Pulverisieren der getrockneten Pellets;
(c) Auflösen der aus Schritt (b) erhaltenen zerkleinerten Pellets mit einer alkalischen Lösung;
(d) Homogenisieren der aus Schritt (c) erhaltenen mit PHA angereicherten alkalischen Lösung;
(e) Trennen des Nicht-PHA-Zellmaterials (NPCM) aus der mit PHA angereicherten Lösung; und
(f) Rückgewinnen des PHA-Granulats aus der mit PHA angereicherten alkalischen Lösung.

2. Verfahren nach Anspruch 1, wobei das PHA aus der Gruppe ausgewählt ist, bestehend aus Poly(hydroxybutyrat-co-hydroxyvalerat) (PHBV) und Polymeren und Copolymeren von hydroxyterminiertem Polyhydroxybutyrat (PHB-OH).

3. Verfahren nach Anspruch 1, wobei die Bakterienzellen PHA-produzierende Mikroorganismen sind.

4. Verfahren nach Anspruch 1, wobei die getrockneten Pellets aus PHA-reichen Bakterienzellen nach der Trennung mittels Zentrifugation produziert werden.

5. Verfahren nach Anspruch 4, wobei die getrockneten Pellets bei ca. 60 °C für ca. 24 Stunden produziert werden.

6. Verfahren nach Anspruch 1, wobei die alkalische Lösung 0,2 molares Natriumhydroxid (NaOH) ist.

7. Verfahren nach Anspruch 6, wobei die alkalische Behandlung der mit PHA angereicherten Lösung mit der alkalischen Lösung für ca. 60 Minuten durchgeführt wird.

8. Verfahren nach Anspruch 1, wobei Schritt (d) bei einer Geschwindigkeit von 8000 UpM durchgeführt wird.

9. Verfahren nach Anspruch 8, wobei die Homogenisierung der mit PHA angereicherten alkalischen Lösung für ca. 18 Minuten durchgeführt wird.

10. Verfahren nach Anspruch 1, wobei das NPCM aus der mit PHA angereicherten alkalischen Lösung durch Zentrifugation getrennt wird.

11. Verfahren nach Anspruch 10, wobei das NPCM durch Zentrifugation bei ca. 3500 UpM für ca. 10 Minuten zweimal getrennt wird.

12. Verfahren nach Anspruch 1, wobei das PHA-Granulat durch zweimaliges Spülen mit entionisiertem Wasser zwischen dem Zentrifugationsverfahren aus der alkalischen Lösung rückgewonnen wird.

13. Verfahren nach Anspruch 1, wobei das PHA-Granulat durch Trocknen produziert wird.

## Revendications

1. Procédé d'extraction et de purification de polyhydroxyalcanoate (PHA) à partir de cellules bactériennes riches en PHA, le procédé comprenant les étapes qui consistent à :
(a) fournir des granulés séchés contenant des cellules bactériennes riches en PHA ;
(b) pulvériser les granulés séchés ;
(c) dissoudre les granulés broyés obtenus à l'étape (b) avec une solution alcaline ;
(d) homogénéiser la solution alcaline enrichie en PHA obtenue à l'étape (c) ;
(e) séparer le matériau cellulaire non-PHA (NPCM) de la solution alcaline enrichie en PHA ; et
(f) récupérer les granulés de PHA à partir de la solution alcaline enrichie en PHA.

2. Procédé selon la revendication 1, dans lequel le PHA est sélectionné dans le groupe constitué du poly(hydroxybutyrate-co-hydroxyvalérate)(PHBV), et de polymères et copolymères de polyhydroxybutyrate à terminaison hydroxy (PHB-OH).

3. Procédé selon la revendication 1, dans lequel les cellules bactériennes sont des micro-organismes qui produisent du PHA.

4. Procédé selon la revendication 1, dans lequel les granulés séchés sont produits à partir de cellules bactériennes riches en PHA après séparation en utilisant un procédé de centrifugation.

5. Procédé selon la revendication 4, dans lequel les granulés séchés sont produits à une température d'environ 60 °C sur une période d'environ 24 heures.

6. Procédé selon la revendication 1, dans lequel la solution alcaline est une solution d'hydroxyde de sodium (NaOH) à 0,2 M.

7. Procédé selon la revendication 6, dans lequel le traitement alcalin de la solution enrichie en PHA en utilisant la solution alcaline est conduit pendant environ 60 minutes.

8. Procédé selon la revendication 1, dans lequel l'étape (d) est conduite à une vitesse de 8 000 trs/min.

9. Procédé selon la revendication 8, dans lequel l'homogénéisation de la solution alcaline enrichie en PHA est conduite pendant environ 18 minutes.

10. Procédé selon la revendication 1, dans lequel le NPCM est séparé de la solution alcaline enrichie en PHA par centrifugation.

11. Procédé selon la revendication 10, dans lequel le NPCM est séparé deux fois par centrifugation à environ 3 500 trs/min pendant environ 10 minutes.

12. Procédé selon la revendication 1, dans lequel les granulés de PHA sont récupérés à partir de la solution alcaline en rinçant deux fois avec de l'eau désionisée à un stade intermédiaire du procédé de centrifugation.

13. Procédé selon la revendication 1, dans lequel les granulés de PHA sont produits par séchage.
